(19) Europäisches Patentamt

European Patent Office

Office européen des brevets

(11) **EP 4 493 056 B1**

(12) **EUROPEAN PATENT SPECIFICATION**

(45) Date of publication and mention
of the grant of the patent:
**17.06.2026 Bulletin 2026/25**

(21) Application number: **23710776.8**

(22) Date of filing: **17.03.2023**

(51) International Patent Classification (IPC):
***A61B 5/103*** *(2006.01)*

(52) Cooperative Patent Classification (CPC):
**A61B 5/1032; A61B 5/441; A61B 5/01;**
A61B 5/14542

(86) International application number:
**PCT/EP2023/056865**

(87) International publication number:
**WO 2023/175132 (21.09.2023 Gazette 2023/38)**

(54) **SYSTEM FOR MONITORING TISSUE HEALTH**

SYSTEM ZUR BEOBACHTUNG DER GESUNDHEIT VON GEWEBE

SYSTÈME POUR LA SURVEILLANCE DE TISSU

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **17.03.2022 EP 22386010**

(43) Date of publication of application:
**22.01.2025 Bulletin 2025/04**

(73) Proprietor: **Pulse Biotech Ltd.
London WC2H 9JQ (GB)**

(72) Inventor: **MARINAKI, Ioanna
Fleet, GU52 8AS (GB)**

(74) Representative: **Gill Jennings & Every LLP
The Broadgate Tower
20 Primrose Street
London EC2A 2ES (GB)**

(56) References cited:
**US-A1- 2022 031 237    US-B2- 10 201 306
US-B2- 10 799 149**

## Description

### FIELD OF THE DISCLOSURE

The disclosure relates to a system and computer-implemented method for monitoring tissue health by performing an objective colour measurement of the tissue and comparing with a baseline colour measurement of the tissue.

### BACKGROUND TO THE DISCLOSURE

This background is provided purely to aid in the understanding of the context in which the disclosure operates. The application makes no admission that any of the subject matter discussed in this background section is prior art.

In medicine, there are a wide variety of circumstances in which the health of a portion of tissue must be monitored over time. One example of this is after a flap transplant, in which tissue from a first donor site is transplanted onto a second recipient site and wherein the re-attaching procedure involves attempting to reconnect the blood vessels to supply blood to the flap. This is such that the flap receives sufficient blood circulation in the second site without relying on the growth of new blood vessels. The hours after such a procedure are vital in determining if the reconnection of the blood supply has been successful as irregularities in venous and/or arterial blood flow to the tissue may cause for example necrosis, requiring subsequent surgery or in some cases limb amputation.

A key indicator in evaluating how the health of a flap is progressing in the hours after a procedure is how the appearance of the tissue changes. For example, if for any reason blood circulation is irregular within portions of the flap, one may observe a whitening of the affected region due to a lack of collateral circulation or a darkening of the affected region due to a pooling of blood. If restricted blood flow occurs for an extended period of time, the ischemia may progress quickly to tissue necrosis and gangrene, leading to a darkening of the affected region. Furthermore, it is common after a flap transplant to have a period of rehabilitation wherein the flap is gradually subjected to a range of venous or arterial pressures by adjusting the vertical position of the flap in relation to the heart. For example, a flap located on the foot may be subjected to an increased venous pressure in a recumbent position by dangling the foot in a lower vertical position than the rest of the body. A change in appearance during this process is also a good indicator that there is irregular circulation of oxygenated blood in the flap.

A change in appearance of a piece of tissue as an indicator to its health is not limited to assessing flaps and also applies to many other conditions. For example, changes in tissue appearance are relevant to conditions resulting in poor circulation such as diabetic foot and Raynaud's disease, some skin conditions presenting visible symptoms and more general problems such as organ failure.

Current techniques for monitoring tissue health rely on one of two mechanisms. In the first mechanism, visual and physical inspections are made by trained medical staff to estimate the health of the tissue. For example, within the first 24/48 hours after a flap transplant, the consultant may recommend an observation to be performed as often as every 30 minutes. Observations may be based on indicators such as visual appearance and temperature to the touch. These inspections require trained medical staff to visit the patient at frequent intervals and use their expertise to assess the health progression of the tissue over time.

In the second mechanism, measurements employing near-IR spectroscopy of the affected tissue can be taken to estimate a tissue health related quantity such as oxygen saturation of the blood. This is performed by exposing the tissue two or more wavelengths of light, comparing their absorption and using this to estimate the proportion of oxygenated to deoxygenated haemoglobin present in the blood. Spectroscopy based on the reflectance of light is the preferred over transmission for this application as it does not require the measurement site to be thin.

One problem with the first mechanism is that a consultant or other medical staff member may not be able to provide an accurate assessment of the health of the tissue via visual or physical inspection as these observations can be subjective based on the circumstances and surrounding environment. For example, observations of the tissue appearance and temperature by touch may be highly subjective due to the lighting of the environment, the initial colouring of the skin and the varying experience of the medical professional. Moreover, this mechanism is inefficient for consultants as they frequently have to spend time observing the tissue, which can be problematic when they have multiple patients to assess and other responsibilities.

One problem with the second mechanism is that oxygen saturation calculations based on reflective spectroscopy require highly expensive specialist equipment and still require medical staff that are specifically trained to operate the device. What is needed is therefore is a means by which the health of a portion of tissue can be monitored objectively that is efficient for both patients and medical staff. US 10799149 B2 discloses computer-based systems and techniques for analysing skin coloration using spectral imaging techniques to determine a medical condition of an individual.

US 2022/031237 A1 discloses systems, methods and computer program products for monitoring vascular perfusion in replanted tissue flaps.

US 10201306 B2 discloses a wound image capture method that uses self colour compensation to improve colour consistency of the captured image and reliability of colour-based wound detection.

## SUMMARY OF THE DISCLOSURE

In a first aspect the disclosure provides a system for monitoring tissue health in accordance with appended claim 1.

Broadly speaking the disclosure provides a systems and computer-implemented methods to monitor and update medical staff of the progression in tissue health via objective colour measurement of the tissue and comparison to a baseline colour of the tissue. Instructions pertaining to the intended usage of said systems and computer-implemented methods may be instigated by a medical professional, for example, indicating the length of time and frequency in which measurements should be performed. A colour sensing module provides a means to objectively measure the colour of a target portion of tissue, a process which can be repeated over time to record the progression in the colour of the tissue. The system is then able, via a processing unit, to analyse a measured colour of the tissue by determining the change in colour of the tissue compared to a baseline colour of the tissue and determine if the change in colour is significant enough to suggest that a degradation of health has occurred. If the change in colour exceeds a threshold, an alert is created indicating that a degradation in the tissue health has occurred. In this way, the system provides a pre-diagnostic tool to inform the user of suspected tissue health degradation and to prompt the user to seek diagnosis from a consultant.

A degradation in the health of tissue making up a transplanted flap is a reliable indicator of the success or failure of a flap transplant procedure. Specifically, the success/failure is dependent on the circulation within the implanted tissue as time progresses after the procedure. Therefore, the present disclosure provides a means to identify failure of the flap transplant and prompt further action from a medical professional before other dangerous side effects take hold.

Objective colour measurement may provide several improvements over traditional visual or physical examinations by removing subjectivity resulting from the lighting of the environment, the initial colouring of the skin and the varying experience of the medical professional. This may provide piece of mind to patients that degradations in their tissue health will not go unnoticed and increase the working efficiency of consultants by only requiring visual or physical examination of the tissue when health degradation is suspected. Furthermore, the system may be used as a training tool for medical staff to improve their visual assessments of tissue health.

The following terms used within this specification should be understood as follows: Tissue: A group of structurally and functionally similar cells and their intercellular material. Tissue may refer to at least any of the 4 basic types of tissue within the human body, namely muscle tissue (e.g. skeletal muscle), nervous tissue (e.g. spinal cord), connective tissue (e.g. dermis and hypodermis) and epithelial tissue (e.g. epidermis).

Flap: A portion of tissue that has been transplanted from a first donor site onto a second recipient site on the human body maintaining an intact vascular supply. A flap may comprise any combination of skin, fascia, muscle, and bone depending on the application.

Flap transplant: A reconstructive surgery in which any type of tissue is removed from a donor site and moved to a recipient site on the body. A flap transplant is different from a skin graft as the tissue is transplanted with an intact blood supply. Flap transplant is intended to cover all types of surgical procedure involving removal and attachment of a flap.

In preferable examples of the disclosure, the target portion of the tissue comprises a flap that has been attached to a recipient site during a flap transplant procedure. In this case, a significant change in colour of the target portion of tissue may indicate irregular venous/arterial blood circulation and/or oxygenation within the flap. There are numerous types of flap transplant surgeries to which the present disclosure may be applied. Examples include: skin flap, pedicle flap, rope flap, advancement flap, sliding flap.

The colour sensing module, processing unit and alerting module may be combined into an integral device or may be provided as one or more separate units connected by a communications interface. For example, the components may be combined into a smart device, for example a smartphone. The colour sensing model is preferably configured to measure a colour of a target portion of tissue and provide measurement data encoding the measured colour to the processing unit. The baseline data preferably encodes a baseline colour of the target portion of tissue. The baseline data is preferably a previous measurement of the target portion of tissue taken with the colour sensing module and stored in a memory. The memory may be local to one or more components of the system, or may be a remote memory accessed, for example, over the internet.

The processing unit may be configured to obtain, from the measurement data, a numerical representation of the measured colour within a colour model, obtain, from the baseline data, a numerical representation of the baseline colour within a colour model and determine a change in the numerical representation of the measurement data relative to the numerical representation of the baseline data. This advantageously provides objective representations of the measured colour, baseline colour and change in colour between the measured colour and baseline colour. The measurement data may comprise the numerical representation of the measured colour or the processing unit may perform an interpretation process on the measurement data to derive the numerical representation of the measured colour. A colour model comprises a model for defining a digital colour. This may also be referred to as a "colour space", where the numerical representation defines the colour within the colour space. For example the numerical representation may comprise a vector, defining a colour within a colour space. In some examples the processing unit may be configured to determine a change between the baseline colour and measured colour by computing a difference between the numerical representations, for example computing a difference between two vectors defining the measured and baseline colours.

The numerical representations of the measurement data and the baseline data may comprise an RGB code representing a colour within the RGB colour model, and the processing unit may be configured to determine a change between an RGB code representing the measured colour of the target portion of tissue and an RGB code representing the baseline colour of the target portion of tissue. This advantageously provides objective representations of the measured colour, baseline colour and change in colour between the measured colour and baseline colour in terms of the widely compatible RGB colour model. The processing unit may be configured to determine a change in one, two or all three of the RGB values of an RGB code. In some examples the processing unit may comprise a change in each value against a respective threshold change in order to determine an alert. The threshold for each of the R, G and B values may differ. In some example the threshold may be defined by a function of the thresholds for each individual value.

The processing unit may be configured to determine a change in RGB values by: determining a difference between one or more of the individual red, green and blue components of the RGB code representing the measured colour of the target portion of tissue and the respective component of the code representing the baseline colour of the target portion of tissue. This advantageously provides a means of calculating the difference between the measured colour and baseline colour in which the change in the individual components of the RGB can be compared to the predetermined threshold independently.

In some embodiments, the processing unit is configured to determine the change in colour of the target portion of tissue by calculating a percentage change in one or more components of the measured colour and baseline colour.

The system comprises a memory and the baseline data may comprise a previous measurement of a colour of the target portion of tissue stored in the memory. This advantageously provides baseline data that is specific to the target portion of tissue and monitoring device in use.

The baseline colour is selected from a plurality of previous measurements of a colour of the target portion of tissue stored in the memory defining a base reference range of the colour of the target portion of tissue. The baseline colour comprises an average of a plurality of previous measurements defining the base reference range of the colour of the target portion of tissue. Using a plurality of previous measurements to select the baseline colour may advantageously reduce the number of situations wherein a degradation in the health of the target portion of tissue has occurred but the change in colour compared to one or more of the previous measurements did not exceed the predetermined threshold.

The processing unit may be configured to set the predetermined threshold based on a measurement of the colour of a healthy portion of tissue of the patient presenting the target portion of tissue. This may advantageously improve the correlation between a change in colour that is greater than the predetermined threshold and a degradation in the health of the target portion of tissue.

The colour sensing module may further comprise one of:

  a photodetector, a red light source, a blue light source and a green light source;
  three or more photodetectors and a white light source;
  an image sensor;
  a colourimeter;
  a spectrophotometer;

In some examples the colour sensing module comprises a light source configured to excite a fluorescent label present in the target portion of tissue and a photodetector configured to detect fluorescent radiation emitted from the label. By providing a fluorescent label in the target portion of tissue, a fluorescence spectrum may be obtained by the photodetector as baseline data and measurement data to reflect a change in colour of the target portion of tissue between the measured colour of the target portion of tissue and the baseline colour of the target portion of tissue. Preferably, the light source is configured to excite a quantum dot based label.

The system may further comprise a temperature sensor configured to measure the temperature of the target portion of tissue or alternatively the processing unit may be configured to determine, from the measurement data, a temperature of the target portion of tissue. The processing unit may be configured to set the predetermined threshold based on a measured temperature of the target portion of tissue. In other examples the processor may be configured to compare a temperature measurement against a baseline temperature measurement and provide an alert with the alerting module when the change in temperature exceeds a threshold. In other examples the processor may be configured to provide an alert when the measured temperature and colour data exhibits a particular combined behaviour. This may advantageously improve the correlation between a change in colour that is greater than the predetermined threshold and a degradation in the health of the target portion of tissue.

Where the colour sensing module comprises an image sensor, the processing unit may be configured to determine a measured colour by one or more of: averaging across the entirety or a portion of the image data received from the image sensor, receive a user selection of a position on the image from which the measured colour should be determined; automatically determine a location of the target tissue portion within the image and determine the measured colour at this location.

The system may further comprise a light source and a separation component, such that when placed in contact with the target portion of tissue, the separation component is configured to separate the target portion of tissue from the colour sensing module, permit propagation of light from the light source between the colour sensing module and target portion of tissue and prevent light from sources external to the system from illuminating the target portion of tissue. This advantageously provides consistency to the illumination of the target portion of tissue between measurements such that the colour measurements of the target portion of tissue are not affected by variable ambient lighting. The separation component preferably comprises a tubular or conical body have an opening at a first end arranged to surround the colour sensing module and light source and an opening at a second, opposite, end arranged to surround the target portion of tissue. In this way the body allows the light source to illuminate the target portion of tissue without the influence of external, ambient light, which is prevented from entering the tubular body, thereby ensuring reproducible lighting conditions and more accurate colour measurement. Preferably, the separation component can be sterilised so that it can be safely placed in contact with the flap tissue

In some examples, the separation component further comprises a lens. This may be, for example, a periscope type lens. The focusing function of the lens may assist the colour sensing module in obtaining higher quality baseline and measurement data.

Preferably, the separation component further comprises a projection, the projection movable between a first position and a second position and arranged such that when the separation component is placed in contact with the target portion of tissue, the projection makes contact with the target portion of tissue when in the first position and does not make contact with the target portion of tissue when in the second position. In this way, the operator of the system is able to measure, using the contact of the projection with the target portion of tissue, a capillary refill time to assess blood flow through the target portion of tissue.

In some examples, the measurement data comprises at least one measurement of the target portion of tissue with the projection in the first position and at least one measurement of the target portion of tissue with the projection in the second position. In these examples, in addition to determining a change in colour between the measurement data and baseline data, the processor may be configured to calculate a capillary refill time based on a change in colour between measurements in the measurement data, for example, by comparing the change in colour to a threshold. Notably, capillary refill is observed as a change in colour of the target portion of tissue from white to red.

The system may further comprise a pulse oximeter or alternatively the processing unit may be configured to determine, from the measurement data, a blood oxygen saturation level of the target portion of tissue. The processor may receive data from the pulse oximeter and provide an alert when the receive data indicates a degradation in tissue health, for example when a threshold is exceeded. The blood oxygen saturation level may also be used to set the predetermined threshold for a colour change indicative of a degradation in the health of the target portion of tissue.

The processor may use combined data from multiple sources selected from: the colour sensing module, a temperature sensor configured to sense the temperature of the tissue portion, a pulse oximeter configured to determine the oxygen saturation level within the target tissue portion, a blood perfusion sensor configured to measure blood perfusion in the target tissue portion, or may infer these additional properties from measurement data collected by the colour sensing module. Additionally, one or more of the data types other than change in colour may be manually provided to the processor from measurements taken by other external means. The processor may be configured to determine a degradation in tissue health based on one or more of these data types. For example the processor may be configured to input the received data into a predetermined function and output an alert when a threshold is exceeded. In other examples the processing unit may be configured to input two or more of the measured data types into a trained machine learning model configured to predict a tissue health score on the basis of the input data measurements.

The colour sensing module, alerting module, memory and processing unit making up the system may be distributed across a plurality of physical devices. Where these are distributed across different devices, the devices may communicate with each other directly, or via the internet.

The processing unit may be provided in a mobile device, the mobile device configured to display the graphical user interface, the interface comprising one or more interactive elements. This advantageously provides an efficient means for a user to control and exchange information with the system.

In some examples, a mobile device does not perform any of the functions colour sensing module, alerting module, memory and processing unit making up the system, but may still receive alerts from the system and display instances of the graphical user interface.

The system may comprising a first entity and a second entity wherein the colour sensing module is provided in the first entity and the processing unit and alerting module are provided in the second entity. Alternatively the system may comprise a standalone entity, the standalone entity, optionally further comprising a display and a communications module. For example the system may comprise a smartphone running software for carrying out the steps performed by the processor. The separation component may attach to the smartphone. In particular the system may comprise a tubular or conical component that attaches to the smartphone so as to enclose the target portion of tissue, the camera and the flash of the smartphone. In this way ambient light is prevented from entering the tubular component and the target portion is reproducibility illuminated by the smartphone, thereby removing variation in measurements due to changes in ambient lighting.

In some examples, the system is further configured to alert, based on a predefined schedule, a user to perform a colour measurement. The schedule is typically part of a post operative plan, digitally stored and accessible to the processing unit. The plan may include a frequency with which measurements should be performed and the duration over which measurements should be performed at a given frequency. Such a post operative plan may be specified to by a medical professional such as the responsible consultant and stored in a local or remote memory.

Preferably, the processing unit is further configured to output an indication as to whether the target portion of tissue has been successfully implanted at the recipient site. This indication may be based on, for example, one or more prior changes in colour of the target portion of tissue.

The invention is defined by the appended claims.

## BRIEF DESCRIPTION OF DRAWINGS

The disclosure is described with reference to the following figures which illustrate, by way of example only, particular embodiments of the disclosure.

Figure 1 illustrates in schematic form a system for monitoring tissue health according to an embodiment

Figure 2 shows a side profile of a system for monitoring tissue health according to a second embodiment

Figure 3 shows a side profile of a system for monitoring tissue health according to a third embodiment

Figure 4 shows a side profile of a system for monitoring tissue health according to a fourth embodiment

Figure 5 shows a side profile of a system for monitoring tissue health according to a fifth embodiment

Figures 6a, 6b and 6c collectively illustrate in schematic form instances of a graphical user interface for controlling a system for monitoring tissue health and navigating previous tissue health records.

Figure 7 illustrates a process for monitoring the health of a target portion of tissue.

Figure 8 illustrates a process for measuring and storing one or more baseline colours of a target portion of tissue.

## DETAILED DESCRIPTION

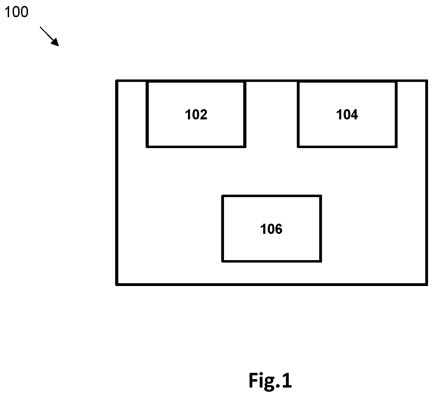

Fig. 1 illustrates in schematic form an exemplary tissue health monitoring system 100 according to an embodiment.

The system 100 is configured to monitor the health of a target portion of tissue implanted at a recipient site as part of a flap transplant procedure. The system 100 includes a colour sensing module 102, a processing unit 104 and alerting module 106.

The system 100 may further include (all not shown) a display, a user input module, a communications interface such as any combination of a Wi-Fi interface, a Bluetooth interface, a cellular network interface or a USB interface, enabling two way communication between the system 100 and a remote computer, a biometric authentication interface such as a fingerprint reader, a temperature sensor configured to measure a temperature range encompassing the range of human body temperature, a pulse oximeter and a separation component, such that when placed in contact with the target portion of tissue, the separation component is configured to separate the target portion of tissue from the colour sensing module 102, permit propagation of light from a light source between the colour sensing module 102 and target portion of tissue, and prevent light from sources external to the system from illuminating the target portion of tissue.

The colour sensing module 102 is configured for measuring a colour of a target portion of tissue and can include any type of sensor capable of outputting a signal indicative of one or more colours in the visible spectrum. Examples include one or more photodetectors or an image sensor such as a CMOS sensor or charge-coupled device, optionally integrated in a camera, colourimeter or spectrophotometer. It will be appreciated that the colour sensing module 102 may include a composite structure that is formed of multiple individual sensors. The colour sensing module 102 may be configured to output a signal indicative of a single colour, a signal indicative of an image comprising a colour at each pixel location or a signal indicative of light intensity across a spectrum of colours in the visible range.

Where the colour sensing module 102 comprises a single photodetector, the colour sensing module 102 may further include an RGB light source capable of emitting each of red, green and blue light. Said light source may be a single RGB LED or individual red, green and blue LEDs. Where the colour sensing module 102 comprises three or more photodetectors or an image sensor, the colour sensing module 102 may further comprise a colour filter array such as a Bayer filter and a white light source such as a white LED. The colour sensing module 102 may further include a lens arrangement or an arrangement of light pipes and/or optical fibres to direct emitted and reflected light. Wherein the sensing portion of the colour sensing module 102 outputs analogue signals, the colour sensing module 102 may further include an analogue to digital converter such that the processing unit 104 is able to read the output signal of the colour sensing module 102.

The colour sensing module 102 may alternatively comprise a light source configured to excite a fluorescent label present in the target portion of tissue and a photodetector configured to detect fluorescent radiation emitted from the label. The fluorescent radiation may not be in a visible range and therefore the photodetector may be sensitive to light from other bands of the electromagnetic spectrum such as near-infrared, infrared or ultraviolet. When a fluorescent label is provided in the target portion of tissue, a fluorescence spectrum may be obtained by the photodetector as baseline data and measurement data to reflect a change in colour of the target portion of tissue between the measured colour of the target portion of tissue and the baseline colour of the target portion of tissue. In these examples, the light source may be configured to excite a quantum dot based label.

The processing unit 104 may include any type of processor making use of any computer architecture. Examples include a microprocessor, an integrated circuit such as a microcontroller or ASIC or a programmable logic device (PLD) such as a FPGA or CPLD. The processing unit 104 may also represent distributed computing capabilities, e.g. a microprocessor and a central processing unit located within a separate entities. The processing unit 104 may be communicatively coupled to a memory such that the processing unit 104 can execute instructions stored in said memory and send data to be stored in said memory.

The processing unit 104 may be configured to perform neuromorphic computing. The processing unit 104 may comprise a neuromorphic architecture and may be coupled with a colour sensing module 102 that may comprise a neuromorphic image sensor such as a dynamic vision sensor to generate input signals for the processing unit 104. The input signals generated by such a dynamic vision sensor may be based on relative changes in the temporal contrast of a given pixel, creating a series of spikes which may be processed by an asynchronous event based spiking neural network to simulate natural learning. The processing unit 104 may be configured to determine an estimated neural plasticity of the target portion of tissue. Neural plasticity refers to the capacity of the nervous system to modify itself, functionally and structurally, in response to experience and injury and as such, the neural plasticity of the newly implanted tissue may additionally indicate a health of the tissue. The determination of neural plasticity may be based on a neural algorithm which evolves over time and takes one or more of the change in colour, temperature, and blood oxygen saturation level as inputs and outputs a percentage of plasticity. This percentage may be in comparison with other areas of tissue.

The processing unit 104 is configured to receive, from the colour sensing module 102, measurement data comprising a measured colour of a target portion of tissue, determine, using the measurement data and baseline data comprising a baseline colour of the target portion of tissue, a change in colour of the target portion of tissue between the measured colour of the target portion of tissue and the baseline colour of the target portion of tissue and output an alert, by the alerting module 106, when the change in colour of the target portion of tissue is greater than a predetermined threshold, indicating a degradation in the health of the target portion of tissue. Alternatively, an alert may be outputted when a combination of the change in colour and one or more of a measured temperature and blood oxygen saturation of a target portion of tissue is greater than a predetermined threshold.

Additionally, when a change in colour or a combination of the change in colour and one or more of a measured temperature and blood oxygen saturation of the target portion of tissue is less than the predetermined threshold, the processing unit 104 may be configured to output an alert, via alerting module 106, indicating that no degradation in the health of the target portion of tissue has occurred.

The processing unit 104 may be further configured to assign tissue metadata to measurement data received from the colour sensing module 102. Said measurement data and tissue metadata may be stored in the memory of the system 100 as a tissue record such that the measurement data and tissue metadata of a given tissue record can be recalled at a later time. A tissue record may be, for example, an entry in a database. Said tissue metadata may be one or more of a unique identifier for each measurement, a tissue name allowing anonymous identification of the portion of tissue, a timestamp of when the measurement was performed, a location of where the measurement was performed, a name of the patient upon which the measurement was performed, a user name of the user who performed the measurement, a name of the supervising consultant.

Additionally, the processing unit may be further configured to output an indication as to whether the target portion of tissue has been successfully implanted at the recipient site. This indication may be based on, for example, one or more prior changes in colour of the target portion of tissue.

The alerting module 106 may include any device configured for outputting an alert, the alert comprising one or more of a local alert for the operator of the device and a remote alert, intended, for example, to remotely alert the consultant responsible for monitoring the health of the target portion of tissue. For example, a visual alert may be provided by a light source or image on a display, an audible alert may be provided by a tone played through a speaker module, a haptic alert may be provided by a vibration motor or a communicative alert may be provided by the transmission of a message or notification to a consultant by a communications interface.

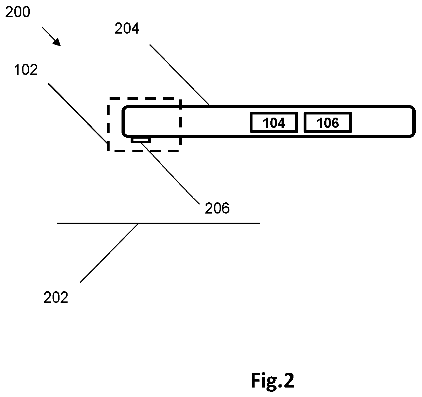

Fig. 2 illustrates an exemplary tissue health monitoring system 200 according to a second embodiment for use in monitoring the health of a target portion of tissue 202. The target portion of tissue 202 can be located at any position on the body and may be, for example, a region of a flap that has been transplanted onto a donor site. Where the target portion of tissue 202 is partially covered by a medical dressing, an observation site on the target portion of tissue 202 may be left uncovered as is commonly practiced to allow visual inspection.

The system 200 comprises a mobile device 204 such as a mobile phone or tablet computer configured such that the colour sensing module 102 is embodied as a camera module 206, the processing unit 104 is embodied by the processor of the mobile device 204 and the alerting module 106 is embodied as any one of a display configured to provide visual feedback, a speaker module configured to audible feedback, a haptics module configured to provide vibration or a communications interface configured to transmit a message or notification to a consultant. The camera module 206 may include a plurality of image sensors and/or an integrated light source such as an LED flash.

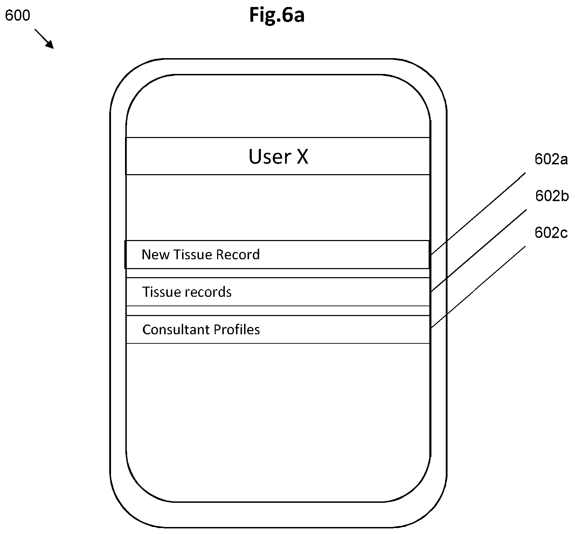

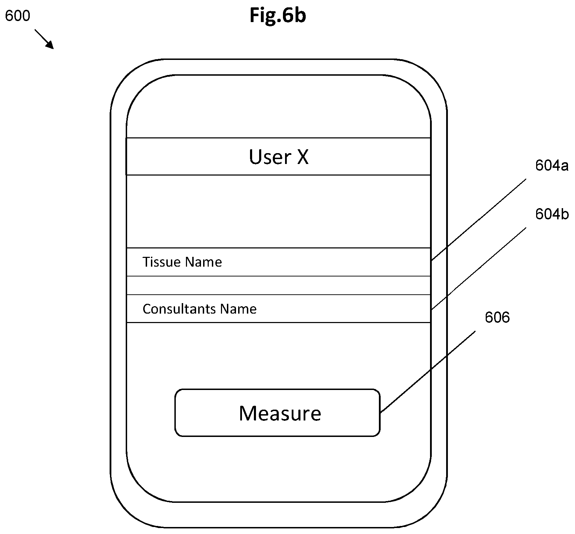

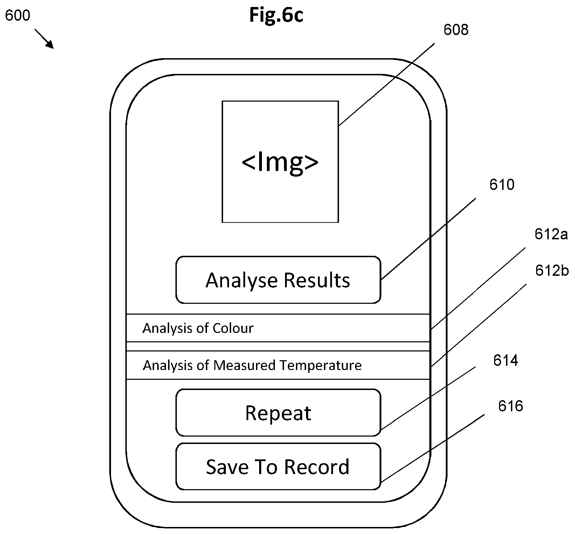

The mobile device 204 preferably includes a communications interface such as any combination of a Wi-Fi interface, a Bluetooth interface, a cellular network interface or a USB interface, enabling two way communication between the mobile device 204 and a remote computer. The mobile device 204 optionally includes one or more of a biometric authentication interface such as a fingerprint reader and a pulse oximeter. All components of the system 200 listed above are conventional and therefore are not described in detail here. The mobile device 204 may be configured to display a graphical user interface 600 to control the function of the system 200. Further details showing the functionality of exemplary instances of the graphical user interface 600 are provided in Figures 6a, 6b and 6c.

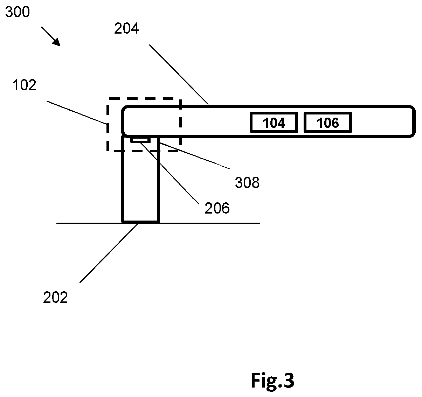

Fig. 3 illustrates an exemplary tissue health monitoring system 300 according to a third embodiment for use in monitoring the health of the target portion of tissue 202.

In addition to the features disclosed in the system 200, the colour sensing module 102 of the system 300 further includes a separation component 308. The separation component 308 is configured to separate the target portion of tissue 202 from the camera module 206 when placed in contact with the target portion of tissue 202. The separation component 308 includes a housing having a first end positioned adjacent to the camera module 206 and a second opposite end positioned extending away from the camera module 206. The separation component 308 is configured such that light is able to propagate between the first and second ends of its housing and may comprise a hollow interior and/or tubular shape. Additional features provided in the interior of the housing of the separation component 308 may include any combination of passive components such as one or more lenses configured to focus light onto the camera module 206 and active components such as additional light sources and a temperature sensor configured to measure a temperature range encompassing the range of human body temperature, wherein the temperature sensor is preferably a non-contact infrared thermometer or thermal imaging camera. Alternatively, in the absence of a pulse oximeter and a temperature sensor, the processing unit 104 may be further configured to infer one or more of the temperature and blood oxygen saturation of the target portion of tissue through measurement data from the camera module 206. Where active components are provided, said active components may be battery powered or powered by the mobile device 204 via a USB interface.

The separation component 308 may be suitable for sterilisation such that it can be safely placed in contact with the target portion of tissue 202 during measurement. To isolate the interior of separation component 308 from the target portion of tissue 202, the separation component 308 may include a transparent or translucent cover placed over the contact point with the target portion of tissue 202. Said cover maybe fixed to the separation component 308 or may be removable.

The separation component 308 may also include within its housing, a projection, the projection movable between a first position and a second position and arranged such that when the separation component is placed in contact with the target portion of tissue, the projection makes contact with the target portion of tissue when in the first position and does not make contact with the target portion of tissue when in the second position. In this way, the operator of the system is able to measure, using the contact of the projection with the target portion of tissue, a capillary refill time to assess blood flow through the target portion of tissue.

When the projection is provided, the user of the system 300 may additionally be able to identify a capillary refill time through a change in colour of the target portion of tissue 202. For example, colour measurements of the target portion of tissue 202 may be performed such that at least one measurement of the target portion of tissue with the projection in the first position and at least one measurement of the target portion of tissue with the projection in the second position, corresponding to applying and removing pressure to the target portion of tissue 202. In these examples, the processing unit 104 may be configured to calculate a capillary refill time based on a change in colour between measurements in the measurement data, for example, by comparing the change in colour to a threshold.

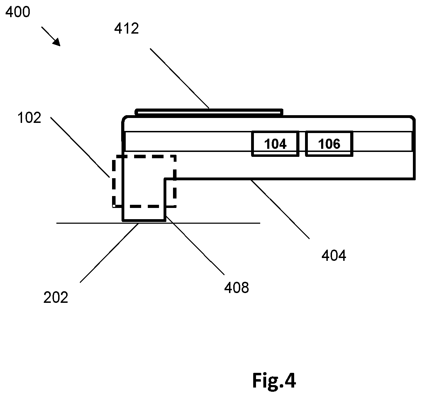

Fig. 4 illustrates an exemplary tissue health monitoring system 400 according to a fourth embodiment for use in monitoring the health of the target portion of tissue 202.

The system 400 is a hand-held standalone measurement device including all of the colour sensing module 102, the processing unit 104 and the alerting module 106 within a housing 404 in any configuration as described in the schematic system 100. The housing 404 may be configured such that a display 412 is provided on a first side of the housing 404 and the colour sensing module 102 is provided on an opposite side of the housing 404 such that the display 412 can be seen by the user during measurement. The display 412 may be configured as a user input module via a touchscreen interface or a separate user input module may be provided.

The system 400 preferably includes a separation component 408 positioned between the colour sensing module 102 and the target portion of tissue 202 configured to separate the target portion of tissue 202 from the colour sensing module 102. The separation component 408 may form part of the housing 404 and in Fig. 4 is shown as a protrusion of the housing 404. Alternatively, the separation component 408 may be a removable attachment to the housing 404, having a first end positioned adjacent to the colour sensing module 102 and a second opposite end positioned extending away from the colour sensing module 102.

The separation component 408 is configured such that light is able propagate between its first and second ends and may comprise a hollow interior and/or tubular shape. Additional features provided in the interior of the separation component 408 may include any combination of passive components such as one or more lenses configured to focus light onto the colour sensing module 102 and active components such as additional light sources. The separation component 408 may be suitable for sterilisation such that it can be safely placed in contact with the target portion of tissue 202 during measurement. To isolate the interior of the separation component 408 from the target portion of tissue 202, the separation component 408 may include a transparent or translucent cover placed over the contact point with the target portion of tissue 202. Said cover maybe fixed to the separation component 408 or may be removable.

The separation component 408 may also include within its housing, a projection, the projection movable between a first position and a second position and arranged such that when the separation component is placed in contact with the target portion of tissue, the projection makes contact with the target portion of tissue when in the first position and does not make contact with the target portion of tissue when in the second position. In this way, the operator of the system is able to measure, using the contact of the projection with the target portion of tissue, a capillary refill time to assess blood flow through the target portion of tissue.

When the projection is provided, the user of the system 400 may additionally be able to identify a capillary refill time through a change in colour of the target portion of tissue 202. For example, colour measurements of the target portion of tissue 202 may be performed such that at least one measurement of the target portion of tissue with the projection in the first position and at least one measurement of the target portion of tissue with the projection in the second position, corresponding to applying and removing pressure to the target portion of tissue 202. In these examples, the processing unit 104 may be configured to calculate a capillary refill time based on a change in colour between measurements in the measurement data, for example, by comparing the change in colour to a threshold.

The system 400 may include a memory suitable for storing measurement data and/or software executable by the processing unit 104. The system 400 preferably includes a communications interface such as any combination of a Wi-Fi interface, a Bluetooth interface, a cellular network interface or a USB interface, enabling two way communication between the system 400 and a remote computer. The system 400 optionally includes one or more of a biometric authentication interface such as a fingerprint reader, a pulse oximeter and a temperature sensor configured to measure a temperature range encompassing the range of human body temperature, wherein the temperature sensor is preferably a non-contact infrared thermometer or thermal imaging camera. Alternatively, in the absence of a pulse oximeter and a temperature sensor, the processing unit 104 may be further configured to infer one or more of the temperature and blood oxygen saturation of the target portion of tissue 202 through measurement data from the colour sensing module 102.

The system 400 may be configured to display, by the display 412, the graphical user interface 600. Further details showing the functionality of exemplary instances of the graphical user interface 600 are provided in Figures 6a, 6b and 6c.

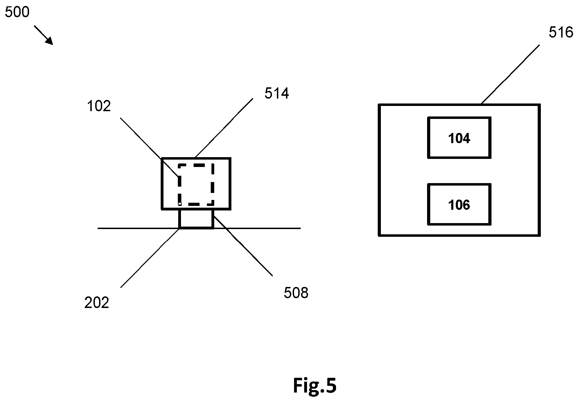

Fig. 5 illustrates an exemplary tissue health monitoring system 500 according to a fifth embodiment for use in monitoring the health of the target portion of tissue 202. The system 500 includes a first entity 514 and a second entity 516 wherein the colour sensing module 102 is provided in the first entity 514 and the processing unit 104 and alerting module 106 are provided in the second entity 516 in any configuration as described in the schematic system 100.

In Fig. 5, the processing unit 104 is shown as a component within the second entity 516. The second entity 516 may also include a memory suitable for storing measurement data and/or software executable by the processing unit 104. Alternatively, the processing unit 104 and memory can be distributed across the first entity 514 and the second entity 516. For example, the first entity 514 may include a microprocessor and a first memory and the second entity 516 may include a central processing unit and a second memory.

The first entity 514 includes the colour sensing module 102 in any configuration as described in the schematic system 100 and may be embodied as a commercially available colour sensor such as a portable colorimeter or spectrophotometer.

The first entity 514 preferably includes a separation component 508 positioned between the colour sensing module 102 and the target portion of tissue 202 configured to separate the target portion of tissue 202 from the colour sensing module 102. The separation component 508 may form part of the housing of the first entity 514 and in Fig. 5 is shown as a protrusion of the first entity 514. Alternatively, the separation component 508 may be a removable attachment to the first entity 514, having a first end positioned adjacent to the colour sensing module 102 and a second opposite end positioned extending away from the colour sensing module 102.

The separation component 508 is configured such that light is able propagate between its first and second ends and may comprise a hollow interior and/or tubular shape. Additional features provided in the interior of the separation component 508 may include any combination of passive components such as one or more lenses configured to focus light onto the colour sensing module 102 and active components such as one or more additional light sources. The separation component 508 may be suitable for sterilisation such that it can be safely placed in contact with the target portion of tissue 202 during measurement. To isolate the interior of the separation component 508 from the target portion of tissue 202, the separation component 508 may include a transparent or translucent cover placed over the contact point with the target portion of tissue 202. Said cover maybe fixed to the separation component 508 or may be removable.

The separation component 508 may also include within its housing, a projection, the projection movable between a first position and a second position and arranged such that when the separation component is placed in contact with the target portion of tissue, the projection makes contact with the target portion of tissue when in the first position and does not make contact with the target portion of tissue when in the second position. In this way, the operator of the system is able to measure, using the contact of the projection with the target portion of tissue, a capillary refill time to assess blood flow through the target portion of tissue.

When the projection is provided, the user of the system 500 may additionally be able to identify a capillary refill time through a change in colour of the target portion of tissue 202. For example, colour measurements of the target portion of tissue 202 may be performed such that at least one measurement of the target portion of tissue with the projection in the first position and at least one measurement of the target portion of tissue with the projection in the second position, corresponding to applying and removing pressure to the target portion of tissue 202. In these examples, the processing unit 104 may be configured to calculate a capillary refill time based on a change in colour between measurements in the measurement data, for example, by comparing the change in colour to a threshold. The first entity 514 may further include one or more of a pulse oximeter and a temperature sensor configured to measure a temperature range encompassing the range of human body temperature, wherein the temperature sensor is preferably a non-contact infrared thermometer or thermal imaging camera. Alternatively, in the absence of a pulse oximeter and a temperature sensor, the processing unit 104 may be further configured to infer one or more of the temperature and blood oxygen saturation of the target portion of tissue 202 through measurement data from the colour sensing module 102.

One or both of the first entity 514 and the second entity 516 may include a communications interface such as any combination of a Wi-Fi interface, a Bluetooth interface, a cellular network interface or a USB interface, enabling two way communication between the first entity 514 and the second entity 516 and/or two way communication between the first entity 514 or second entity 516 and a remote computer.

The first entity 514 and/or second entity 516 may include a display and a user input module. The second entity 516 may be embodied, for example, as a mobile device or a computer. The second entity 516 may be configured to display the graphical user interface 600. Further details showing the functionality of exemplary instances of the graphical user interface 600 are provided in Figures 6a, 6b and 6c.

Figures. 6a, 6b and 6c collectively illustrate in schematic form instances of a graphical user interface 600 for controlling system for monitoring tissue health and navigating stored tissue records. The instances of the graphical user interface 600 shown are examples to aid in the understanding of the disclosure.

The graphical user interface 600 may be displayed via the execution of software stored in a local memory such as a mobile application or as part of a web browser application. The graphical user interface 600 may be configured to retrieve and display tissue records stored in a local memory or retrieve said records from a remote computer via communications interface. The graphical user interface 600 may conform to the ISO/TS 82304-2 standard for health and wellness apps.

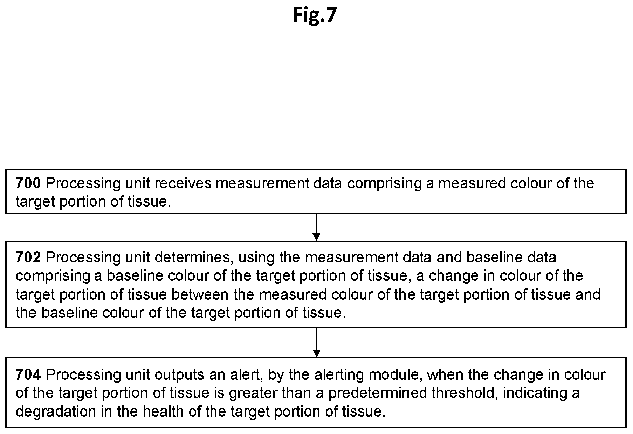

Upon launching the graphical user interface 600, the user may be required in a first instance of the graphical user interface 600 (not shown) to create a user profile or fulfil a means of authentication to gain access to a previously created user profile. This authentication may, for example, be a set of credentials or biometric authentication such as a fingerprint scan. The creation of a new user profile may include the user providing a means of authentication and further may include providing contact information to be assigned to the user profile. Once authenticated, the user may be able to access further instances of the graphical user interface 600 and be granted permissions to access stored tissue records. The user profile may also be associated with a post operative plan as described in more detail below in relation to Figure 7. In such examples, the application executed to display the graphical user interface 600 may also be configured provide alerts to the patient or medical staff based upon the post operative plan associated with a user profile. This may be via the graphical user interface 600, through push notifications, or by remote communication means such as an alert message sent via SMS or other communication protocols. In these examples, the alert may be presented to a device of one or more of the responsible consultant and the patient.

The instance shown in Fig. 6a illustrates a menu presented to an example user, user X, designed to aid in the navigation of the graphical user interface 600.

Interactive elements 602a - 602c are buttons enabling a user X to create a new tissue record, access a list of tissue records and access a list of consultant profiles.

Upon selecting the button 602a, user X may be presented with the instance of the graphical user interface 600 shown in Fig. 6b to create a new tissue record for the target portion of tissue 202.

Upon selecting the button 602b, user X may be presented in an instance of graphical user interface the 600 with a list of stored tissue records. For example, if the profile of user X is a user profile that belongs to a nurse in a hospital environment, the list of tissue records retrieved may include one record for each different instance of the target portion of tissue 202 that were measured across different patients whilst signed into the user X profile. Each element of said list of tissue records may be interactive such that when a tissue record is selected, user X may be able to view any stored measurement data and tissue metadata related to the selected instance of the target portion of tissue 202. Furthermore, from within a tissue record belonging to an instance of the target portion of tissue 202, user X may be able to select to record a new measurement of the same instance of the target portion of tissue 202. When storing a new measurement of a previously recorded instance of the target portion of tissue 202, user X may be presented with the instance of the graphical user interface 600 shown in Fig. 6b with the elements 604a and 604b pre-filled using the corresponding tissue metadata.

Similarly, upon selecting the button 602c, user X may be presented in an instance of the graphical user interface 600 a list of consultant profiles. Each element of said list of consultant profiles may be selectable to view contact details and any tissue records assigned to each consultant.

In Fig. 6b, the elements 604a and 604b illustrate text fields enabling user X to enter a tissue name and consultants name responsible for monitoring the health of the target portion of tissue 202. The content of said fields may be pre-filled if user X has selected to perform a measurement of a previously recorded instance of the target portion of tissue 202 or blank and editable if user X has selected to create a new tissue record for a new instance of the target portion of tissue 202. In this case, the text provided in the fields 604a and 604b may be appended to tissue metadata and stored in the new tissue record for the new instance of the target portion of tissue 202.

The element 606 is a button that when pressed may instruct the processing unit 104 to execute the measurement process of a colour of the target portion of tissue 202 such as shown in Fig. 7. Wherein a temperature sensor is provided, the element 610 may also trigger a measurement of the temperature of the target portion of tissue 202. Wherein a pulse oximeter is provided, the element 610 may also trigger a measurement of the blood oxygen saturation of the target portion of tissue 202. Alternatively, in the absence of a pulse oximeter and a temperature sensor, the element 610 may also trigger the processing unit 104 to infer one or more of the temperature and blood oxygen saturation of the target portion of tissue 202 through measurement data from the colour sensing module 102.

Wherein one or more of a temperature sensor and pulse oximeter is not provided, the graphical user interface 600 may include additional text fields such that user X can provide a measured temperature and/or a blood oxygen saturation recorded with an external measurement device manually to the processing unit 104.

The instance shown in Fig. 6c illustrates a layout presented to user X immediately after a measurement of the target portion of tissue 202 has been performed. The elements 610 and 612a may only be displayed if the measurement just performed was of a previously recorded instance of the target portion of tissue 202 with baseline data already stored in memory pertaining to said instance of the target portion of tissue 202.

The element 608 is an image or non-editable text box that may display an image of the target portion of tissue 202 wherein the colour sensing module includes the camera module 206, display a numerical representation of the measured colour, display a visual sample of the measured colour or display a spectrum of measured colours recorded in the measurement of the target portion of tissue 202. The element 608 may be interactive such that when selected, the image or non-editable text box is enlarged. Wherein the element 608 contains an image of the target portion of tissue 202, the graphical user interface 600 may provide editing functions such as a cropping tool for the image.

The element 610 is a button that triggers the analysis of the measurement of the target portion of tissue 202 including the determination of a change in colour of the target portion of tissue 202 between the measured colour of the target portion of tissue 202 and the baseline colour of the target portion of tissue 202 and outputting an alert, by the alerting module 106, when the change in colour of target portion of the tissue 202 is greater than a predetermined threshold, indicating a degradation in the health of the target portion of tissue 202. Alternatively, an alert may be outputted when a combination of the change in colour and one or more of a measured temperature and blood oxygen saturation of the target portion of tissue 202 is greater than a predetermined threshold. Additionally, when a change in colour or a combination of the change in colour and one or more of a measured temperature and blood oxygen saturation of the target portion of tissue is less than the predetermined threshold, the processing unit 104 may be configured to output an alert, via alerting module 106, indicating that no degradation in the health of the target portion of tissue has occurred.

The element 612a is display field that is an embodiment of the alerting module 106, providing a visual alert to user X to indicate if the change in colour of target portion of the tissue 202 is greater than the predetermined threshold, indicating a degradation in the health of the target portion of tissue 202. One example of such a visual alert is a green thumbs up icon when the predetermined threshold is not exceeded and a red thumbs down icon when the predetermined threshold is exceeded.

The element 612b is a display field configured to visually alert user X to indicate if the measured temperature of the target portion of tissue 202 is outside the predetermined range of temperatures for the target portion of tissue 202. Again, one example of such a visual alert is a green thumbs up icon when the measured temperature is deemed healthy and a red thumbs down icon when the measured temperature is deemed unhealthy.

The graphical user interface may further include a display field (not shown) configured to visually alert user X to indicate if the blood oxygen saturation of the target portion of tissue 202 is outside the range of healthy blood oxygen saturation levels of the target portion of tissue 202. Again, one example of such a visual alert is a green thumbs up icon when the measured temperature is deemed healthy and a red thumbs down icon when the measured temperature is deemed unhealthy.

The processing unit may be configured to automatically alert the assigned consultant by a communications interface configured to transmit a message or notification that either a degradation in health has occurred or no degradation in health has occurred. This may be based on solely change in colour or a combination of change in colour with measured temperature and/or blood oxygen saturation of the target portion of tissue 202.

The element 614 is a button enabling user X to repeat a measurement of the displayed instance of the target portion of tissue 202 and may trigger an identical process as the element 606.

The element 616 is a button enabling user X to save the measurement data, results of the analysis triggered by the element 610 and tissue metadata to a tissue record such that the tissue record may be recalled at a later time.

A process for monitoring the health of the target portion of tissue 202 is described in Fig. 7.

In preparation for measurement, the colour sensing module 102 is positioned such that its sensor can perform a measurement of the target portion of tissue 202. The distance required between the target portion of tissue 202 and the colour sensing module 102 is dependent on the configuration of the colour sensing module 102. For example, wherein the colour sensing module 102 is embodied as the camera module 206, the camera module 206 may be positioned such that the target portion of tissue 202 falls within its field of view. Wherein a separation device is provided, the colour sensing module 102 may be positioned such that the separation device is in contact with the target portion of tissue 202.

At step 700, the processing unit receives measurement data comprising a measured colour of the target portion of tissue 202. The processing unit 104 may be instructed to trigger the colour sensing module 102 to perform a measurement by an interaction through the graphical user interface 600 such as selecting the button 606.

The colour measurement process and composition of the resulting measurement data is dependent on the configuration of the colour sensing module 102. For example, if the colour sensing module 102 comprises a single photodetector and an RGB light source, the measurement process may include illuminating the target portion of tissue 202 with each of red, green and blue light in succession and recording the output from the photodetector for each illumination. If the colour sensing module 102 includes 3 or more photodetectors or an image sensor, the sensor output may be recorded during a single illumination of the target portion of tissue 202 by a white light source either integrated in the system or provided by ambient lighting.

The measurement data may take any form that comprises information pertaining to one or more colour of the target portion of tissue 202 that is readable by the processing unit 104. For example, the measurement data may comprise a digital signal or an analogue signal such as a series of voltages converted to digital by an analogue to digital converter included in the colour sensing module 102.

If the colour sensing module 102 is embodied as an image sensor, said measurement data may comprise a plurality of different colours corresponding to the colour at each pixel location. To extract a single colour of the target portion of tissue 202 from an image, the processing unit 104 may perform further processing of said measurement data. Said further processing may be performed automatically, such as determining an average or most prominent colour across the pixels of the image, or performed based on user input to the graphical interface 600. For example, user X may crop an image of the target portion of tissue 202 to a decreased number of pixels, and instruct the processing unit 104 to determine an average or most prominent colour over the remaining pixels. Alternatively, user X may select a specific pixel from the image from which they wish the colour to be recorded from or the processing unit 104 may perform more complex image colour extraction techniques such as employing a trained machine learning algorithm to intelligently select a colour from an image.

The measured colour of the target portion of tissue 202 may be expressed as a numerical representation or colour code within a colour model. Such a colour model may be, for example, an additive colour model such as the RGB colour model, a subtractive colour model such as the CMYK/CMY colour model, a colour model based on a cylindrical transform such as HSV/HSL or a colour model based upon the human perception of colours such as the tristimulus values of CIEXYZ and any corresponding transforms.

After receiving measurement data, the processing unit 104 may assign tissue metadata to said measurement data and store them as a tissue record in memory such that a user can efficiently retrieve said measurement data at a later time. Said memory may be localised within the measurement device or may be remote and accessed via a communications module.

At step 702, the processing unit 104 determines, using the measurement data and baseline data comprising a baseline colour of the target portion of tissue 202, a change in colour of the target portion of tissue 202 between the measured colour of the target portion of tissue 202 and the baseline colour of the target portion of tissue 202. The baseline data and baseline colour may take any form described above in relation to the measured data and measured colour.

The baseline data comprises a previous measurement comprising a colour of the target portion of tissue 202 and the processing unit 104 may use tissue metadata to retrieve any stored baseline data from memory that are related to the particular instance of the target portion of tissue 202 that is to be measured. Said tissue metadata may be implicitly provided to the processing unit 104 via user X navigating through the graphical user interface 600 to a tissue record belonging to the instance of the target portion of tissue 202 to be measured.

The change in colour may be determined as a numerical metric or set of numerical metrics representing a mathematical difference between the digital representations of the measured colour and baseline colour, or may be a metric modified to suit the human perception of colours.

Colour models such as those described in relation to step 700 are generally described as numerical positions within a 3 or 4 dimensional coordinate system. In this configuration, one method of determining the change between the measured colour and baseline colour is calculating the Euclidean distance between said colours in their respective coordinate system. Furthermore, the standard Euclidean distance formula may be modified by adding correction factors and/or weighting each component of the colour representations differently to better suit human perception of the colour difference. An example of this is the CIE $\Delta E_{94}^*$ and $\Delta E_{00}^*$ perceived colour difference metrics. Alternatively, the change in colour may also be calculated as the percentage change in one or more components of the measured colour and baseline colour.

Said change in colour may be calculated based on a metric taking into account the change in all components of the digital colour representations, or may take into account changes only in a subset of the components of the digital colour representations. For example, in the RGB colour model, the processing unit 104 may be configured to determine a difference between one or more of the individual red, green and blue components of the RGB code representing the measured colour of the target portion of tissue 202 and the respective component of the code representing the baseline colour of the target portion of tissue 202.

For example, in the RGB colour model, the measured colour and baseline colour may be represented as a hexadecimal RGB code (hex code) comprising three hexadecimal numbers describing the proportions of red, green and blue present within the colour. The processing unit 104 may be configured to determine the change between the measured colour and baseline colour in this format by forming a composite numerical value representing the RGB value by concatenating the hexadecimal representations of two or three of the red, green and blue components of the RGB value into a single decimal representation. For example, the RGB colours (135, 26, 24) and (152, 30, 16) may be expressed as the hex codes #871A18 and #981E10 respectively. If said hex codes are treated as single hexadecimal numbers and concatenated into single decimal representations, the resulting decimal representations are 8854040 and 9969168 respectively. The processing unit 104 may then be configured to use these decimal representations in determining a difference between the composite numerical value representing the measured colour and the composite numerical value representing the baseline colour, either calculating an absolute change or a relative change such as a percentage change.

Alternatively, the change in colour may be calculated by any suitably trained machine learning algorithm configured to take measurement data and baseline data as inputs and return a metric or set of metrics representing the change in colour of the target portion of tissue 202.

Optionally, where a plurality of baseline colours defining a base reference range are retrieved from memory, the change in colour calculation may be performed by processing unit 104 using the measured colour and each different baseline colour to generate a plurality of changes in colour. Alternatively, the change in colour calculation may be performed between the measured colour and the average of the retrieved baseline colours to generate a single change in colour. If a plurality of changes in colour are calculated by the processing unit 104, the baseline colour may be selected from the plurality of previous measurements of a colour of the target portion of tissue stored in the memory, such that the change in colour of the target portion of tissue 202 is maximised.

In step 704, the processing unit 104 outputs an alert via the alerting module 106 if the change in colour is greater than a predetermined threshold, indicating a degradation in health of the target portion of tissue. Alternatively, an alert may be outputted when a combination of the change in colour and one or more of a measured temperature and blood oxygen saturation of the target portion of tissue 202 is greater than a predetermined threshold. Additionally, when a change in colour or a combination of the change in colour and one or more of a measured temperature and blood oxygen saturation of the target portion of tissue is less than the predetermined threshold, the processing unit 104 may be configured to output an alert, via alerting module 106, indicating that no degradation in the health of the target portion of tissue has occurred.

Alternatively, the processing unit may be configured to input two or more of the measured data types into a trained machine learning model configured to predict a tissue health score on the basis of the input data measurements.

The form of the predetermined threshold is dependent on how the change in colour between the measured colour and baseline colour is represented in step 702. The predetermined threshold may be a single value or set of values that may be set based on factors such as the sensitivity of the colour sensing module 102, the recommendations of the consultant responsible for the target portion of tissue 202, a measurement of the colour of a healthy portion of tissue of the patient presenting the target portion of tissue 202 and a measured temperature of the target portion of tissue 202 or a measured blood oxygen saturation of the target portion of tissue 202. Furthermore, the predetermined threshold may be adjustable from within the graphical user interface 600, allowing a user to choose the sensitivity. Alternatively, the predetermined threshold may be set by any suitably trained machine learning algorithm configured to take any combination of factors affecting the setting of the predetermined threshold as input.

The alert outputted by the alerting module 106 is dependent on the embodiment of the alerting module 106. Examples of such an alert include a visual alert via a light source. This may include, for example, illuminating a previously unilluminated light source, flashing a light source or changing the colour of a light source. A visual alert may also be provided via a display and may form part of the graphical user interface 600. An audible alert may be provided by a speaker module such a beep or spoken instructions, a haptic alert may be provided by a vibration motor and a communicative alert may be provided by a communications interface configured to transmit a message or notification to a remote consultant.

Such a message or notification may be, for example, an e-mail, SMS message or push notification.

Following completion of the health monitoring process of Fig. 7, the processing unit 104 may update the tissue record corresponding to the measured instance of the target portion of tissue 202 by storing the results of the process in memory, enabling the retrieval of the results pertaining to the particular instance of the target portion of tissue 202 at a later time.

The process of Fig. 7 may be performed at regular intervals to monitor the progression in health of the target portion of tissue 202. For example, where the target portion of tissue 202 is a region of a flap, the process may be performed at least during the first 24-48 hours following the procedure as this is the time frame in which it is most likely to observe a severe degradation in the health due to an irregular rate of perfusion. The process may be further performed during a period of rehabilitation after the procedure to monitor the progression in health over time. After a medical procedure such as a flap transplant where it is common for monitoring to take place at time intervals for a certain duration after the procedure, a post operative plan may be created defining the schedule for future measurements. The plan may be digitally stored and accessible by the processing unit This plan may include a frequency with which measurements should be performed and the duration over which measurements should be performed at a given frequency. Such a post operative plan may be specified to by a medical professional such as the responsible consultant and may be linked to the patient, for example, via the user profile described in relation to Figures 6a-6c.

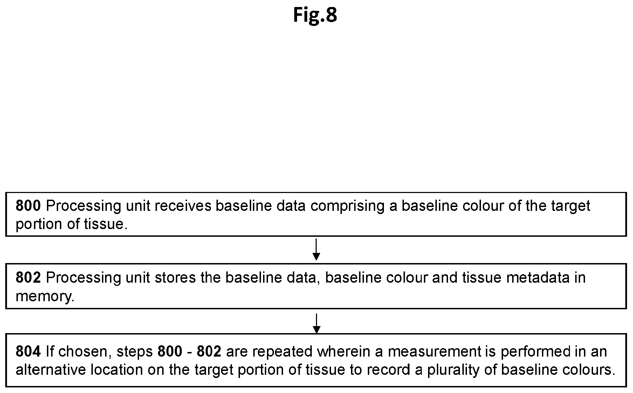

Fig. 8 illustrates a process for measuring and storing one or more baseline colours of the target portion of tissue 202. A baseline colour may comprise a measurement of a colour of the target portion of tissue 202 performed following a significant event within the treatment of the target portion of tissue 202. The baseline data may be collected by the surgeon immediately following the flap transplant procedure.

At step 800, the processing unit 104 receives baseline data comprising a baseline colour of the target portion of tissue in equivalent way to the measurement data in step 700. The processing unit 104 may be instructed to trigger the colour sensing module 102 to perform a baseline measurement by an interaction through the graphical user interface 600 such as selecting the button 606.

At step 802, the processing unit 104 is configured to store baseline data, the digital representation of the baseline colour and corresponding tissue metadata pertaining to the target portion of tissue 202 in memory. Said memory may be localised within the measurement device or may be remote and accessed via a communications module.

At step 804, the user may choose to record more than one baseline measurement of the target portion of tissue 202. Multiple baseline measurements may recorded at the same location on the target portion of tissue 202 or at different locations on the target portion of tissue 202, defining a base reference range. A repeated measurement may be actioned, for example, by selecting the button 614 in the graphical user interface 600.

Either prior to or upon completion of the measurement of one or more baseline colours of the target portion of tissue 202, the user, preferably the consultant responsible for monitoring the health of the target portion of tissue, may be prompted to provide to the processing unit 104 a plan detailing instructions for the future use to monitor the target portion of tissue 202. Said plan may be stored in memory and may include details such as the time period over which subsequent measurements should be performed and the frequency with which the subsequent measurements should be performed during said time period.

Based on the plan, the processing unit 104 may be configured to notify the user and/or consultant responsible, for example via the graphical user interface 600 or via the alerting module 106, at the times a subsequent measurement is due to be performed. Alternatively or additionally, a subsequent measurement may be performed at any time at the request of the patient. A subsequent measurement may comprise, for example, the process described in Fig. 7.

As used herein, the term "non-transitory computer-readable media" includes all tangible, computer-readable media, including, without limitation, non-transitory computer storage devices, including, without limitation, volatile and non-volatile media, and removable and non-removable media such as a firmware, physical and virtual storage, CD-ROMs, DVDs, and any other digital source such as a network or the Internet, as well as yet to be developed digital means, with the sole exception being a transitory, propagating signal.

**Claims**

1.  A system (100) for monitoring the health of a target portion of tissue (202) implanted at a recipient site as part of a flap transplant procedure, the system (100) comprising:

    a colour sensing module (102) for measuring a colour of the target portion of tissue (202);
    an alerting module (106) for outputting an alert indicating a suspected degradation in tissue health;
    a memory;
    a display; and
    a processing unit (104) configured to:

    receive, from the colour sensing module (102), measurement data comprising a measured colour of the target portion of tissue (202);
    determine, using the measurement data and baseline data comprising a baseline colour of the target portion of tissue (202), a change in colour of the target portion of tissue (202) between the measured colour of the target portion of tissue (202) and the baseline colour of the target portion of tissue (202); and
    output an alert, by the alerting module (106), when the change in colour of the target portion of tissue (202) is greater than a predetermined threshold, indicating a degradation in the health of the target portion of tissue (202),
    wherein the processor is configured to determine the baseline colour by:

    launching, on the display, a graphical user interface prompting a user to fulfil a means of authentication associated with a user profile;
    authenticating the user, the authenticating comprising receiving the means of authentication;
    after authenticating the user, receiving, from the colour sensing module (102), a plurality of successive baseline measurements of a colour of the target portion of tissue (202), wherein the plurality of baseline measurements are repeated measurements defining a base reference range of a current colour of the target portion of tissue (202), wherein the baseline measurements are each triggered by a button presented in the graphical user interface; storing the plurality of baseline measurements in the memory; and averaging the plurality of baseline measurements to obtain the baseline colour.

2.  The system (100) according to claim 1 wherein the processing unit (104) is configured to:

    obtain, from the measurement data, a numerical representation of the measured colour within a colour model
    obtain, from the baseline data, a numerical representation of the baseline colour within a colour model; and
    determine a change in the numerical representation of the measured colour relative to the numerical representation of the baseline colour.

3. The system (100) according to claim 2 wherein the numerical representations of the measurement data and the baseline data comprise an RGB code representing a colour within the RGB colour model, and the processing unit (104) is configured to determine a change between an RGB code representing the measured colour of the target portion of tissue (202) and an RGB code representing the baseline colour of the target portion of tissue (202).

4. The system (100) according to claim 3 wherein the processing unit (104) is configured to determine a change in RGB values by:
determining a difference between one or more of the individual red, green and blue components of the RGB code representing the measured colour of the target portion of tissue (202) and the respective component of the code representing the baseline colour of the target portion of tissue (202).

5. The system (100) according to any preceding claim, wherein the processing unit (104) is configured to determine the change in colour of the target portion of tissue (202) by calculating a percentage change in one or more components of the measured colour and baseline colour.

6. The system (100) according to any preceding claim wherein the processing unit (104) is configured to set the predetermined threshold based on a measurement of the colour of a healthy portion of tissue of the patient presenting the target portion of tissue (202).

7. The system (100) according to any preceding claims wherein the colour sensing module (102) comprises one of:

   a photodetector, a red light source, a blue light source and a green light source;
   three or more photodetectors and a white light source.

8. The system (100) according to any one of claims 1 to 7 wherein the colour sensing module (102) comprises a light source configured to excite a fluorescent label present in the target portion of tissue (202) and a photodetector configured to detect fluorescent radiation emitted from the label.

9. The system (100) according to claim 8 wherein light source is configured to excite a quantum dot based label.

10. The system (100) according to any preceding claims wherein the colour sensing module (102) comprises an image sensor.

11. The system (100) according to claim 10 wherein the processing unit (104) is configured to receive image data from the image sensor and determine the measurement data comprising a measured colour of the target tissue portion by one or more of:

   determining an average colour across the entirety or a portion of the image data received from the image sensor;
   receive a user selection of a position on the image from which the measured colour should be determined and determine a colour at the selected position;
   automatically determine a location of the target tissue portion within the image and determine the measured colour at the determined location.

12. The system (100) according to any preceding claim further comprising a temperature sensor configured to measure the temperature of the target portion of tissue (202).

13. The system (100) according to any one of claims 1 to 11 wherein the processing unit (104) is configured to determine, from the measurement data, a temperature of the target portion of tissue (202).

14. The system (100) according to any preceding claim further comprising a pulse oximeter configured to measure a blood oxygen saturation level within the target portion of tissue (202).

15. The system (100) according to any one of claims 1 to 13 wherein the processing unit (104) is configured to determine, from the measurement data, a blood oxygen saturation level of the target portion of tissue (202).

16. The system (100) according to any preceding claim wherein the processing unit (104) is configured to set the predetermined threshold based on one or more of a measured temperature and a blood oxygen saturation level of the target portion of tissue (202).

17. The system (100) according to any preceding claim further comprising a light source and a separation component (308), such that when placed in contact with the target portion of tissue (202), the separation component (308) is configured to:

   separate the target portion of tissue (202) from the colour sensing module (102);
   permit propagation of light from the light source between the colour sensing module (102) and target portion of tissue (202); and
   prevent light from sources external to the system (100) from illuminating the target portion of tissue (202).

**18.** The system (100) according to claim 17 wherein the separation component (308) further comprises a lens.

**19.** The system (100) according to claim 17 or claim 18 wherein the separation component (308) further comprises a projection, the projection movable between a first position and a second position and arranged such that when the separation component (308) is placed in contact with the target portion of tissue (202), the projection makes contact with the target portion of tissue (202) when in the first position and does not make contact with the target portion of tissue (202) when in the second position.

**20.** The system (100) according to claim 19 wherein the measurement data comprises at least one measurement of the target portion of tissue (202) with the projection in the first position and at least one measurement of the target portion of tissue (202) with the projection in the second position.

**21.** The system (100) according to any preceding claim wherein the processing unit (104) is provided in a mobile device (204), the mobile device configured to display the graphical user interface.

**22.** The system (100) according to any one of claims 1 to 20 wherein the colour sensing module (102), alerting module (106) are comprised in a standalone device (400).

**23.** The system (100) according to any preceding claim further configured to alert, based on a predefined schedule, a user to perform a colour measurement.

**24.** The system (100) according to any preceding claim, wherein the processing unit (104) is further configured to output an indication as to whether the target portion of tissue (202) has been successfully implanted at the recipient site.

**25.** A computer-implemented method for monitoring the health of a target portion of tissue (202) implanted at a recipient site as part of a flap transplant procedure, the method comprising:

receiving, from a colour sensing module (102), measurement data comprising a measured colour of the target portion of tissue (202);
determining, using the measurement data and baseline data comprising a baseline colour of the target portion of tissue (202), a change in colour of the target portion of tissue (202) between the measured colour of the target portion of tissue (202) and the baseline colour of the target portion of tissue (202);
outputting an alert, by an alerting module (106), when the change in colour of the target portion of tissue (202) is greater than a predetermined threshold, indicating a degradation in the health of the target portion of tissue (202),
wherein determining the baseline colour comprises:

launching, on a display, a graphical user interface prompting a user to fulfil a means of authentication associated with a user profile;
authenticating the user, the authenticating comprising receiving the means of authentication;
after authenticating the user, receiving, from the colour sensing module (102), a plurality of successive baseline measurements of a colour of the target portion of tissue (202), wherein the plurality of baseline measurements are repeated measurements defining a base reference range of a current colour of the target portion of tissue (202), wherein the baseline measurements are each triggered by a button presented in the graphical user interface;
storing the plurality of baseline measurements in the memory; and
averaging the plurality of baseline measurements to obtain the baseline colour.

**Patentansprüche**

**1.** System (100) zum Überwachen der Gesundheit eines Zielgewebeabschnitts (202), der an einer Empfängerstelle als Teil eines Lappentransplantationseingriffs implantiert wird, wobei das System (100) umfasst:

ein Farberfassungsmodul (102) zum Messen einer Farbe des Zielgewebeabschnitts (202);
ein Alarmierungsmodul (106) zum Ausgeben eines Alarms, der eine vermutete Verschlechterung der Gewebegesundheit angibt;
einen Speicher;
eine Anzeige; und
eine Verarbeitungseinheit (104), die konfiguriert ist, zum:

Empfangen von dem Farberfassungsmodul (102) von Messdaten, die eine gemessene Farbe des Zielgewebeabschnitts (202) umfassen;
Bestimmen unter Verwendung der Messdaten und von Ausgangsdaten, die eine Ausgangsfarbe des Zielgewebeabschnitts (202) umfassen, einer Farbänderung des Zielgewebeabschnitts (202) zwischen der gemessenen Farbe des Zielgewebeabschnitts (202) und der Ausgangsfarbe des Zielgewebeabschnitts (202); und

Ausgeben eines Alarms durch das Alarmierungsmodul (106), wenn die Farbänderung des Zielgewebeabschnitts (202) größer als eine vorbestimmte Schwelle ist, was eine Verschlechterung der Gesundheit des Zielgewebeabschnitts (202) angibt,

wobei der Prozessor konfiguriert ist, um die Ausgangsfarbe zu bestimmen durch:

> Starten auf der Anzeige einer grafischen Benutzeroberfläche, die den Benutzer auffordert, ein einem Benutzerprofil zugeordnetes Authentifizierungsmittel zu erfüllen;
> Authentifizieren des Benutzers, wobei das Authentifizieren das Empfangen des Authentifizierungsmittels umfasst; nach dem Authentifizieren des Benutzers, Empfangen einer Mehrzahl von aufeinanderfolgenden Ausgangsmessungen einer Farbe des Zielgewebeabschnitts (202) von dem Farberfassungsmodul (102), wobei die Mehrzahl von Ausgangsmessungen wiederholte Messungen sind, die einen Basisreferenzbereich einer aktuellen Farbe des Zielgewebeabschnitts (202) definieren, wobei die Ausgangsmessungen jeweils durch eine Schaltfläche ausgelöst werden, die in der grafischen Benutzeroberfläche dargestellt wird;
> Speichern der Mehrzahl von Ausgangsmessungen in dem Speicher; und
> Mittelwertbilden der Mehrzahl von Ausgangsmessungen, um die Ausgangsfarbe zu erhalten.

2. System (100) nach Anspruch 1, wobei die Verarbeitungseinheit (104) konfiguriert ist, zum:

> Erhalten aus den Messdaten einer numerischen Darstellung der gemessenen Farbe innerhalb eines Farbmodells;
> Erhalten aus den Ausgangsdaten einer numerischen Darstellung der Ausgangsfarbe innerhalb eines Farbmodells; und
> Bestimmen einer Änderung der numerischen Darstellung der gemessenen Farbe im Vergleich zu der numerischen Darstellung der Ausgangsfarbe.

3. System (100) nach Anspruch 2, wobei die numerischen Darstellungen der Messdaten und der Ausgangsdaten einen RGB-Code umfassen, der eine Farbe innerhalb des RGB-Farbmodells darstellt, und die Verarbeitungseinheit (104) dazu konfiguriert ist, eine Änderung zwischen einem RGB-Code, der die gemessene Farbe des Zielgewebeabschnitts (202) darstellt, und einem RGB-Code, der die Ausgangsfarbe des Zielgewebeabschnitts (202) darstellt, zu bestimmen.

4. System (100) nach Anspruch 3, wobei die Verarbeitungseinheit (104) dazu konfiguriert ist, eine Änderung der RGB-Werte zu bestimmen durch:
Bestimmen einer Differenz zwischen einer oder mehreren der einzelnen Rot-, Grün- und Blaukomponenten des RGB-Codes, der die gemessene Farbe des Zielgewebeabschnitts (202) darstellt, und der jeweiligen Komponente des Codes, der die Ausgangsfarbe des Zielgewebeabschnitts (202) darstellt.

5. System (100) nach einem der vorhergehenden Ansprüche, wobei die Verarbeitungseinheit (104) dazu konfiguriert ist, die Farbänderung des Zielgewebeabschnitts (202) durch Berechnen einer prozentualen Änderung in einer oder mehreren Komponenten der gemessenen Farbe und der Ausgangsfarbe zu bestimmen.

6. System (100) nach einem der vorhergehenden Ansprüche, wobei die Verarbeitungseinheit (104) dazu konfiguriert ist, die vorbestimmte Schwelle auf Grundlage einer Messung der Farbe eines gesunden Gewebeabschnitts des Patienten, die den Zielgewebeabschnitt (202) darstellt, einzustellen.

7. System (100) nach einem der vorhergehenden Ansprüche, wobei das Farberkennungsmodul (102) eines umfasst von:

> einem Fotodetektor, einer roten Lichtquelle, einer blauen Lichtquelle und einer grünen Lichtquelle;
> drei oder mehr Fotodetektoren und einer weißen Lichtquelle.

8. System (100) nach einem der Ansprüche 1 bis 7, wobei das Farberfassungsmodul (102) eine Lichtquelle, die dazu konfiguriert ist, eine fluoreszierende Markierung anzuregen, die in dem Zielgewebeabschnitt (202) vorhanden ist, und einen Fotodetektor umfasst, der dazu konfiguriert ist, die von der Markierung emittierte Fluoreszenzstrahlung zu erkennen.

9. System (100) nach Anspruch 8, wobei die Lichtquelle dazu konfiguriert ist, eine auf Quantenpunkten basierende Markierung anzuregen.

10. System (100) nach einem der vorhergehenden Ansprüche, wobei das Farberkennungsmodul (102) einen Bildsensor umfasst.

11. System (100) nach Anspruch 10, wobei die Verarbeitungseinheit (104) dazu konfiguriert ist, Bilddaten von dem Bildsensor zu empfangen und die Messdaten, die eine gemessene Farbe des Zielgewebeabschnitts umfassen, durch eines oder mehrere zu bestimmen von:

   Bestimmen einer durchschnittlichen Farbe über die Gesamtheit oder einen Abschnitt der von dem Bildsensor empfangenen Bilddaten;
   Empfangen einer Benutzerauswahl einer Position auf dem Bild, von der aus die gemessene Farbe bestimmt werden sollte, und Bestimmen einer Farbe an der ausgewählten Position;
   automatisches Bestimmen einer Position des Zielgewebeabschnitts innerhalb des Bildes und Bestimmen der gemessenen Farbe an der bestimmten Stelle.

12. System (100) nach einem der vorhergehenden Ansprüche, das ferner einen Temperatursensor umfasst, der dazu konfiguriert ist, die Temperatur des Zielgewebeabschnitts (202) zu messen.

13. System (100) nach einem der Ansprüche 1 bis 11, wobei die Verarbeitungseinheit (104) dazu konfiguriert ist, aus den Messdaten eine Temperatur des Zielgewebeabschnitts (202) zu bestimmen.

14. System (100) nach einem der vorhergehenden Ansprüche, das ferner ein Pulsoximeter umfasst, das dazu konfiguriert ist, einen Blutsauerstoffsättigungsniveau innerhalb des Zielgewebeabschnitts (202) zu messen.

15. System (100) nach einem der Ansprüche 1 bis 13, wobei die Verarbeitungseinheit (104) dazu konfiguriert ist, aus den Messdaten ein Blutsauerstoffsättigungsniveau des Zielgewebeabschnitts (202) zu bestimmen.

16. System (100) nach einem der vorhergehenden Ansprüche, wobei die Verarbeitungseinheit (104) dazu konfiguriert ist, die vorbestimmte Schwelle auf Grundlage eines oder mehrerer einer gemessenen Temperatur oder eines Blutsauerstoffsättigungsniveaus des Zielgewebeabschnitts (202) einzustellen.

17. System (100) nach einem der vorhergehenden Ansprüche, das ferner eine Lichtquelle und eine Trennkomponente (308) umfasst, sodass die Trennkomponente (308), wenn sie in Kontakt mit dem Zielgewebeabschnitt (202) platziert wird, konfiguriert ist, zum:

   Trennen des Zielgewebeabschnitts (202) von dem Farberfassungsmodul (102);
   Zulassen der Ausbreitung von Licht von der Lichtquelle zwischen dem Farberfassungsmodul (102) und dem Zielgewebeabschnitt (202); und
   Verhindern, dass Licht von Quellen außerhalb des Systems (100) den Zielgewebeabschnitt (202) beleuchtet.

18. System (100) nach Anspruch 17, wobei die Trennkomponente (308) ferner eine Linse umfasst.

19. System (100) nach Anspruch 17 oder 18, wobei die Trennkomponente (308) ferner einen Vorsprung umfasst, wobei der Vorsprung zwischen einer ersten Position und einer zweiten Position beweglich ist und derart angeordnet ist, dass, wenn die Trennkomponente (308) in Kontakt mit dem Zielgewebeabschnitt (202) platziert wird, der Vorsprung den Zielgewebeabschnitt (202) berührt, wenn er sich in der ersten Position befindet, und den Zielgewebeabschnitt (202) nicht berührt, wenn er sich in der zweiten Position befindet.

20. System (100) nach Anspruch 19, wobei die Messdaten wenigstens eine Messung des Zielgewebeabschnitts (202) mit dem Vorsprung in der ersten Position und wenigstens eine Messung des Zielgewebeabschnitts (202) mit dem Vorsprung in der zweiten Position umfassen.

21. System (100) nach einem der vorhergehenden Ansprüche, wobei die Verarbeitungseinheit (104) in einer mobilen Vorrichtung (204) bereitgestellt ist, wobei die mobile Vorrichtung dazu konfiguriert ist, die graphische Benutzeroberfläche anzuzeigen.

22. System (100) nach einem der Ansprüche 1 bis 20, wobei das Farberfassungsmodul (102), das Alarmierungsmodul (106) in einer eigenständigen Vorrichtung (400) enthalten sind.

23. System (100) nach einem der vorhergehenden Ansprüche, das ferner dazu konfiguriert ist, einen Benutzer auf Grundlage eines vordefinierten Zeitplans zu alarmieren, eine Farbmessung durchzuführen.

24. System (100) nach einem der vorhergehenden Ansprüche, wobei die Verarbeitungseinheit (104) ferner dazu konfiguriert ist, eine Angabe darüber auszugeben, ob der Zielgewebeabschnitt (202) erfolgreich an der Empfängerstelle implantiert worden ist.

25. Computerimplementiertes Verfahren zum Überwachen der Gesundheit eines Zielgewebeabschnitts (202), der an einer Empfängerstelle als Teil eines Lappentransplantationseingriffs implantiert wird, wobei das Verfahren umfasst:

Empfangen von einem Farberfassungsmodul (102) von Messdaten, die eine gemessene Farbe des Zielgewebeabschnitts (202) umfassen; Bestimmen unter Verwendung der Messdaten und von Ausgangsdaten, die eine Ausgangsfarbe des Zielgewebeabschnitts (202) umfassen, einer Farbänderung des Zielgewebeabschnitts (202) zwischen der gemessenen Farbe des Zielgewebeabschnitts (202) und der Ausgangsfarbe des Zielgewebeabschnitts (202); Ausgeben eines Alarms durch ein Alarmierungsmodul (106), wenn die Farbänderung des Zielgewebeabschnitts (202) größer als eine vorbestimmte Schwelle ist, was eine Verschlechterung der Gesundheit des Zielgewebeabschnitts (202) angibt, wobei das Bestimmen der Ausgangsfarbe umfasst:

Starten auf einer Anzeige einer grafischen Benutzeroberfläche, die den Benutzer auffordert, ein einem Benutzerprofil zugeordnetes Authentifizierungsmittel zu erfüllen; Authentifizieren des Benutzers, wobei das Authentifizieren das Empfangen des Authentifizierungsmittels umfasst; nach dem Authentifizieren des Benutzers, Empfangen einer Mehrzahl von aufeinanderfolgenden Ausgangsmessungen einer Farbe des Zielgewebeabschnitts (202) von dem Farberfassungsmodul (102), wobei die Mehrzahl von Ausgangsmessungen wiederholte Messungen sind, die einen Basisreferenzbereich einer aktuellen Farbe des Zielgewebeabschnitts (202) definieren, wobei die Ausgangsmessungen jeweils durch eine Schaltfläche ausgelöst werden, die in der grafischen Benutzeroberfläche dargestellt wird; Speichern der Mehrzahl von Ausgangsmessungen in dem Speicher; und Mittelwertbilden der Mehrzahl von Ausgangsmessungen, um die Ausgangsfarbe zu erhalten.

## Revendications

1. Système (100) servant à surveiller la santé d'un fragment tissulaire cible (202) implanté au niveau d'un site receveur dans le cadre d'une procédure de reconstruction par lambeau, le système (100) comprenant :

un module de détection des couleurs (102) servant à mesurer la couleur du fragment tissulaire cible (202) ;
un module d'alerte (106) servant à émettre une alerte indicatrice d'une dégradation suspectée de la santé du tissu ;
une mémoire ;
un dispositif d'affichage ; et
une unité de traitement (104) configurée pour :

recevoir, du module de détection des couleurs (102), des données de mesure comprenant une couleur mesurée du fragment tissulaire cible (202) ;
identifier, en utilisant les données de mesure et des données de départ comprenant la couleur de départ du fragment tissulaire cible (202), un changement de la couleur du fragment tissulaire cible (202) entre la couleur mesurée du fragment tissulaire cible (202) et la couleur de départ du fragment tissulaire cible (202) ; et
émettre une alerte au moyen du module d'alerte (106) quand le changement de la couleur du fragment tissulaire cible (202) dépasse un seuil prédéterminé, indiquant une dégradation de la santé du fragment tissulaire cible (202),
où le processeur est configuré pour déterminer la couleur de départ comme suit :

lancement, sur le dispositif d'affichage, d'une interface utilisateur graphique invitant un utilisateur à remplir un moyen d'authentification associé au profil de l'utilisateur ;
authentification de l'utilisateur, l'authentification comprenant la réception du moyen d'authentification ;
après authentification de l'utilisateur, réception d'une pluralité de mesures de départ successives de la couleur du fragment tissulaire cible (202) transmises par le module de détection des couleurs (102), où la pluralité de mesures de départ sont des mesures répétées définissant une gamme de référence de départ pour une couleur actuelle du fragment tissulaire cible (202), les mesures de départ étant chacune déclenchées au moyen d'une touche disponible dans l'interface utilisateur graphique ;
stockage de la pluralité de mesures de départ dans la mémoire ; et
calcul de la moyenne des mesures de départ pour obtenir la couleur de départ.

2. Système (100) selon la revendication 1, où l'unité de traitement (104) est configurée pour :

> obtenir, à partir des données de mesure, une représentation numérique de la couleur mesurée dans un espace colorimétrique ;
> obtenir, à partir des données de départ, une représentation numérique de la couleur de départ dans un espace colorimétrique ; et
> identifier un changement de la représentation numérique de la couleur mesurée par rapport à la représentation numérique de la couleur de départ.

3. Système (100) selon la revendication 2, où les représentations numériques des données de mesure et des données de départ comprennent un code RGB représentant une couleur dans l'espace colorimétrique RGB et où l'unité de traitement (104) est configurée pour identifier un changement entre un code RGB qui représente la couleur mesurée du fragment tissulaire cible (202) et un code RGB qui représente la couleur de départ du fragment tissulaire cible (202).

4. Système (100) selon la revendication 3, où l'unité de traitement (104) est configurée pour identifier un changement des valeurs RGB comme suit :
identification d'une différence entre une ou plusieurs des composantes individuelles rouge, vert et bleu du code RGB qui représente la couleur mesurée du fragment tissulaire cible (202) et la composante correspondante du code RGB qui représente la couleur de départ du fragment tissulaire cible (202).

5. Système (100) selon l'une quelconque des revendications précédentes, où l'unité de traitement (104) est configurée pour identifier le changement de couleur du fragment tissulaire cible (202) en calculant le pourcentage de changement de une ou plusieurs composantes de la couleur mesurée et de la couleur de départ.

6. Système (100) selon l'une quelconque des revendications précédentes, où l'unité de traitement (104) est configurée pour définir le seuil prédéterminé sur la base d'une mesure de la couleur d'une portion de tissu sain du patient qui comprend le fragment tissulaire cible (202).

7. Système (100) selon l'une quelconque des revendications précédentes, où le module de détection des couleurs (102) comprend un entre :

> un photodétecteur, une source de lumière rouge, une source de lumière bleue et une source de lumière verte ;
> trois ou plus photodétecteurs et une source de lumière blanche.

8. Système (100) selon l'une quelconque des revendications 1 à 7, où le module de détection des couleurs (102) comprend une source de lumière configurée pour exciter un marqueur fluorescent présent dans le fragment tissulaire cible (202) et un photodétecteur configuré pour détecter le rayonnement fluorescent émis par le marqueur.

9. Système (100) selon la revendication 8, où la source de lumière est configurée pour exciter un marqueur à boîte quantique.

10. Système (100) selon l'une quelconque des revendications précédentes, où le module de détection des couleurs (102) comprend un capteur d'images.

11. Système (100) selon la revendication 10, où l'unité de traitement (104) est configurée pour recevoir des données d'image à partir du capteur d'images et déterminer les données de mesure comprenant la couleur mesurée du fragment tissulaire cible par le biais d'une ou de plusieurs approches consistant à :

> déterminer une couleur moyenne sur l'ensemble ou sur une partie des données d'image transmises par le capteur d'images ;
> recevoir une sélection utilisateur d'une position sur l'image à partir de laquelle la couleur mesurée devrait être déterminée et déterminer la couleur à la position sélectionnée ;
> identifier automatiquement un emplacement sur le fragment tissulaire cible dans l'image et déterminer la couleur mesurée à l'emplacement identifié.

12. Système (100) selon l'une quelconque des revendications précédentes comprenant en outre un capteur de température configuré pour mesurer la température du fragment tissulaire cible (202).

13. Système (100) selon l'une quelconque des revendications 1 à 11, où l'unité de traitement (104) est configurée pour déterminer la température du fragment tissulaire cible (202) à partir des données de mesure.

14. Système (100) selon l'une quelconque des revendications précédentes comprenant en outre un oxymètre de pouls configuré pour mesurer le taux de saturation en oxygène du sang dans le fragment tissulaire cible (202).

15. Système (100) selon l'une quelconque des revendications 1 à 13, où l'unité de traitement (104) est configurée pour déterminer le taux de saturation en oxygène du sang dans le fragment tissulaire cible (202) à partir des données de mesure.

**16.** Système (100) selon l'une quelconque des revendications précédentes, où l'unité de traitement (104) est configurée pour définir le seuil prédéterminé sur la base de la mesure de la température et/ou du taux de saturation en oxygène du sang dans le fragment tissulaire cible (202).

**17.** Système (100) selon l'une quelconque des revendications précédentes comprenant en outre une source de lumière et un composant de séparation (308), de sorte que quand placé en contact avec le fragment tissulaire cible (202), le composant de séparation (308) est configuré pour :

séparer le fragment tissulaire cible (202) du module de détection des couleurs (102) ; permettre la propagation de la lumière émise par une source de lumière entre le module de détection des couleurs (102) et le fragment tissulaire cible (202) ; et empêcher la lumière émanant de sources extérieures au système (100) d'illuminer le fragment tissulaire cible (202).

**18.** Système (100) selon la revendication 17, où le composant de séparation (308) comprend en outre une lentille.

**19.** Système (100) selon la revendication 17 ou la revendication 18 où le composant de séparation (308) comprend une projection, la projection pouvant être déplacée entre une première position et une seconde position et étant agencée de sorte que quand le composant de séparation (308) est placé en contact avec le fragment tissulaire cible (202), la projection vient en contact avec le fragment tissulaire cible (202) quand elle est dans la première position et ne vient pas en contact avec le fragment tissulaire cible (202) quand elle est dans la seconde position.

**20.** Système (100) selon la revendication 19, où les données de mesure comprennent au moins une mesure du fragment tissulaire cible (202) alors que la projection est dans la première position et au moins une mesure du fragment tissulaire cible (202) alors que la projection est dans la seconde position.

**21.** Système (100) selon l'une quelconque des revendications précédentes où l'unité de traitement (104) est fournie dans un appareil mobile (204), l'appareil mobile étant configuré pour afficher l'interface utilisateur graphique.

**22.** Système (100) selon l'une quelconque des revendications 1 à 20, où le module de détection des couleurs (102), le module d'alerte (106) sont compris dans un appareil autonome (400).

**23.** Système (100) selon l'une quelconque des revendications précédentes qui est en outre configuré pour alerter un utilisateur pour qu'une mesure de la couleur soit effectuée sur la base d'un programme prédéfini .

**24.** Système (100) selon l'une quelconque des revendications précédentes, où l'unité de traitement (104) est en outre configurée pour produire une indication signalant si le fragment tissulaire cible (202) a ou non été implanté avec succès au niveau du site receveur.

**25.** Procédé mis en œuvre par ordinateur servant à surveiller la santé d'un fragment tissulaire cible (202) implanté au niveau d'un site receveur dans le cadre d'une procédure de reconstruction par lambeau, le procédé comprenant les étapes qui consistent à :

recevoir, d'un module de détection des couleurs (102), des données de mesure comprenant une couleur mesurée du fragment tissulaire cible (202) ; identifier, en utilisant les données de mesure et des données de départ comprenant la couleur de départ du fragment tissulaire cible (202), un changement de la couleur du fragment tissulaire cible (202) entre la couleur mesurée du fragment tissulaire cible (202) et la couleur de départ du fragment tissulaire cible (202) ; émettre une alerte au moyen d'un module d'alerte (106) quand le changement de la couleur du fragment tissulaire cible (202) dépasse un seuil prédéterminé, indiquant une dégradation de la santé du fragment tissulaire cible (202), où la détermination de la couleur de départ comprend :

le lancement, sur un dispositif d'affichage, d'une interface utilisateur graphique invitant un utilisateur à remplir un moyen d'authentification associé au profil de l'utilisateur ; l'authentification de l'utilisateur, l'authentification comprenant la réception du moyen d'authentification ; après authentification de l'utilisateur, la réception d'une pluralité de mesures de départ successives de la couleur du fragment tissulaire cible (202) transmises par le module de détection des couleurs (102), où la pluralité de mesures de départ sont des mesures répétées définissant une gamme de référence de départ pour une couleur actuelle du fragment tissulaire cible (202), les mesures de départ étant chacune déclenchées au moyen d'une touche disponible dans l'interface utilisateur graphique ;
le stockage de la pluralité de mesures de départ dans la mémoire ; et
le calcul de la moyenne des mesures de départ pour obtenir la couleur de départ.

Fig.1

Fig.2

**Fig.3**

EP 4 493 056 B1

Fig.4

516

104

106

514

102

508

202

500

**Fig.5**

**Fig.6a**

600

| User X |
| --- |
|  |
| New Tissue Record |
| Tissue records |
| Consultant Profiles |

602a

602b

602c

**Fig.6b**

Fig.6c

**Fig.7**

**700** Processing unit receives measurement data comprising a measured colour of the target portion of tissue.

**702** Processing unit determines, using the measurement data and baseline data comprising a baseline colour of the target portion of tissue, a change in colour of the target portion of tissue between the measured colour of the target portion of tissue and the baseline colour of the target portion of tissue.

**704** Processing unit outputs an alert, by the alerting module, when the change in colour of the target portion of tissue is greater than a predetermined threshold, indicating a degradation in the health of the target portion of tissue.

## Fig.8

**800** Processing unit receives baseline data comprising a baseline colour of the target portion of tissue.

↓

**802** Processing unit stores the baseline data, baseline colour and tissue metadata in memory.

↓

**804** If chosen, steps **800** - **802** are repeated wherein a measurement is performed in an alternative location on the target portion of tissue to record a plurality of baseline colours.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10799149 B2
- US 2022031237 A1

- US 10201306 B2